# EUROPÄISCHE PATENTSCHRIFT

(11) **EP 1 401 569 B1**
(45) Veröffentlichungstag und Bekanntmachung des Hinweises auf die Patenterteilung: **27.09.2006**
(21) Anmeldenummer: 02740299.9
(22) Anmeldetag: 25.04.2002
(51) Int. Cl.: B01J 19/00

(54) **VORRICHTUNG ZUR GLEICHZEITIGEN MULTIPLEN UND HOCHPARALLELEN SYNTHESE**
DEVICE FOR SIMULTANEOUS MULTIPLE AND HIGH PARALLEL SYNTHESIS
DISPOSITIF POUR LA SYNTHESE MULTIPLE ET HAUTEMENT PARALLELE SIMULTANEE

(30) Priorität: 25.06.2001 DE 10131088
(43) Veröffentlichungstag der Anmeldung: 31.03.2004
(73) Patentinhaber: Peptides & Elephants Gmbh, 14558 Bergholz-Rehbrücke (DE)
(72) Erfinder: KREUZER, Oliver, Johannes, 10823 Berlin (DE); HESSENIUS, Carsten, 12049 Berlin (DE); GRÖTZINGER, Carsten, 10115 Berlin (DE); JOHANNSON, Volker, 22303 Hamburg (DE); ROEDER, Ralph-Th., 14478 Potsdam (DE)
(74) Vertreter: Wablat, Wolfgang
(86) Internationale Anmeldenummer: PCT/DE2002/001556
(87) Internationale Veröffentlichungsnummer: WO 2003/000403

(56) Entgegenhaltungen:
- EP-A- 0 355 266
- EP-A- 1 088 585
- US-A- 1 686 188
- US-A- 5 551 487

## Beschreibung

Die Erfindung betrifft eine Vorrichtung zur gleichzeitigen multiplen und hochparallelen Synthese von Verbindungen auf der Basis der Festphasensynthese, insbesondere für die Peptidsynthese.

Für die organische Festphasensynthese von Peptiden, bei der gegenüber den klassischen Syntheseverfahren an einen funktionalen Träger viele verschiedene Synthesebausteine (Aminosäuren) in einem Prozessschritt aneinander gekuppelt werden, sind bereits zahlreiche Vorrichtungen bekannt, die eine automatisierte gleichzeitige und parallele Synthese von über 100 unterschiedlichen Peptiden ermöglichen - WO 98/35753; DE 196 02 464 A1.

Diese bekannten Vorrichtungen sind nach dem Prinzip von Pipettierrobotern aufgebaut, wobei die Reagenzien und Lösemittel aus entsprechenden Vorratsgefäßen über eine oder mehrere Kanülen, die an einen in x-, y - und z-Achse bewegbaren Arm der Synthesevorrichtung befestigt sind, zu den Reaktionskammern transportiert und dem in den Reaktionskammern befindlichen Trägermaterial für die durchzuführende Synthese zudosiert werden.
Zur Herstellung von Biomolekülen schlägt WO 98/35753 eine Vorrichtung für die automatisierte chemische Synthese vor, bei der die Verteilung der Reagenzien mit einer Mehrfachkanüle erfolgt. Diese,Mehrfachkanüle muß nach Verteilung jedes Synthesebausteines gespült werden, um Kontaminationen zwischen den Reagenzien sicher auszuschließen. Für eine Synthese mit 48 verschiedenen Peptiden und einer Kettenlänge von 10 Synthesebausteinen beträgt die reine Spülzeit 8h, wobei für einen Spülvorgang eine Spülzeit von 1 min. in Ansatz gebracht werden muß. Für 96 Peptide beträgt die Spülzeit beispielsweise 16h, für 384 Peptiden 64h und für 1536 Peptide sogar 256h. Eine hohe Parallelisierung der Synthese ist somit nicht erreichbar.

Die Verwendung von Syntheseplatten, die von Mikrotiterplatten mit einem 96er oder 384er Lochraster abgeleitet sind, ist ebenfalls beschrieben. Sie sind auf einen Ventilblock aufgesetzt, über den die unverbrauchten Reagenzien, die Spülflüssigkeiten und die Abspaltlösungen für die temporären Schutzgruppen mittels einer Vakuumpumpe abgesaugt werden. Hierzu sind die Reaktionskammer, wie beispielsweise aus DE 197 44 549 A1 ersichtlich, bodenseitig durch eine poröse Membran verschlossen, damit die Reaktionsflüssigkeiten und verbrauchten Reagenzien abgesaugt werden können.

Trotz der wesentlichen Vorteile, die mit diesen Einrichtungen für eine automatisierte, zeitgleiche, multiple und parallele Synthese verbunden sind, besteht ein entscheidender Nachteil darin, dass die verschiedenen Reagenzien durch gleiche Schlauchsysteme und Kanülen geleitet werden und die Gefahr von Kreuzkontaminationen besteht. Um Kontaminationen auszuschließen, wird bei den bekannten Geräten das gesamte System mit großen Volumina an Spülmitteln gespült. Neben den Materialkosten für das Spülmittel verzögert sich durch die notwendigen Spülvorgänge die Synthesezeit erheblich. Ein weiterer Nachteil besteht darin, dass die bekannten Einrichtungen aufgrund der offenen Systeme der Pipettierautomaten eine Schutzgasatmosphäre für voraktivierte Reagenzien nicht zulassen. Daraus resultieren eine erhöhte Instabilität und damit ein erhöhter Reagenzienverbrauch und die Notwendigkeit zum manuellen Reagenzienaustausch.

Eine weitere Synthesevorrichtung ist aus DE 198 18 999 A1 bekannt. Das Trägermaterial für die Synthese ist auf einer rotierenden Trommel oder eine rotierende Scheibe aufgetragen, wobei im Trägermaterial sogenannte Spots zur Aufnahme der Reagenzien ausgebildet sind. Über einen linear zur Trommelachse oder radial zur Scheibenachse verstellbaren Dosierkopfhalter, in den mehrere Dosierköpfe in Form von Pipetten angeordnet sind, werden die Reagenzien, die durch Schläuche aus einem Vorrat entnommen werden, dem jeweiligen Spot dosiert zugeführt. Auch diese Einrichtung ist im wesentlichen mit den gleichen Nachteilen, wie vorstehend dargelegt, behaftet. Außerdem erschweren die notwendigen Schlauchverbindungen eine leichte und einfache Handhabung. Bei dieser Lösung entfallen zwar die Spülschritte für die Verteilung der Synthesebausteine, die Synthese wird jedoch nicht in Reaktionskammern durchgeführt, sondern finden auf einem durchgehenden planaren Träger, beispielsweise Folie, Papier, Glas etc. statt. Synthesen in einem größeren Maßstab sind nicht möglich, wobei für die Synthese keine flexiblen Trägersysteme eingesetzt werden können, sondern nur Träger die planar einsetzbar sind.

Zum Stand der Technik gehören ferner Tuscheschreiber für Zeichengeräte und sogenannte Markierungsstifte, mit denen eine im Körper des Tuscheschreibers oder des Markierungsstiftes bevorratete Flüssigkeit nach Aufsetzen auf eine Unterlage dosiert ausgebracht werden kann. In Bereichen der Labortechnik bzw. für die organische Festphasen- und Flüssigphasensynthese und andere chemische/biochemische Untersuchungs- und Analyseverfahren sind derartige Ausrüstungen nicht bekannt.

Der Erfindung liegt die Aufgabe zugrunde, eine verbesserte Vorrichtung für eine automatisierte Synthese von Verbindungen zu entwickeln, die die bisher notwendigen Spülvorgänge nach der Reagenzienverteilung und einem Wechsel der Reagenzien vermeidet und die Zuführung, Dosierung und Reagenzienbevorratung für jeden einzelnen Synthesebaustein in einem Vorrichtungselement zusammenfasst. Außerdem sollen die Synthese, das Abspalten der Verbindungen vom Syntheseharz und das Entschützen der Seitenketten, was bisher nur in getrennten Vorrichtungen vorgenommen werden konnte, mit einer einzigen, voll automatisierten Vorrichtung realisierbar sein.

Erfindungsgemäß wird die Aufgabe durch eine Vorrichtung nach den im Anspruch 1 angegebenen Merkmalen gelöst. Vorteilhafte Weiterbildungen der Erfindung ergeben sich aus den Merkmalen der Unteransprüche 2 bis 8.

Durch die Erfindung wird eine Vorrichtung vorgeschlagen, die gänzlich ohne Dilutoren und Schlauchsysteme für die Zuführung und dosierte Abgabe der Synthesebausteine arbeitet. Für die einzelnen Synthesebausteine sind jeweils separate Synthesestifte vorgesehen, die in einer Halterung der Synthesevorrichtung für die Synthese bereitgestellt sind und aus dieser Halterung vom Greifarm der Vorrichtung erfasst und aufgenommen werden, um eine dosierte Menge des Bausteines auf das in einer Reaktionskammer einer Syntheseplatte befindliche Trägermaterial abzugeben. Reagenzienreservoir und Dosiervorrichtung bilden somit eine in sich geschlossene Einheit. Dadurch entfallen sämtliche Spülvorgänge und die damit verbundenen Nachteile, die bisher bei einem Wechsel der Synthesebausteine und der Reagenzienverteilung notwendig waren.

Die Syntheseplatte ist modular aufgebaut und besteht aus zehn einzelnen Platten, die die Reaktionskammern beinhalten. Diese sind im 96, 384 oder 1536 Lochraster analog zu den Mikrotiterplatten unterteilt. Nach den weiteren Merkmalen der Erfindung sind die Reaktionskammern der Syntheseplatten öffnungsseitig mit einem permeablen Material, beispielsweise mit einer Fritte verschlossen. Unterhalb des Ventilblockes sind Probenplatten für die Aufnahme der nach Abspaltreaktion gelösten Proben angeordnet, die mit einer Absauganlage verbunden sind. Mit der vorgeschlagenen Vorrichtung für die Festphasensynthese ist sowohl die Synthese als auch die Abspaltung der gewonnenen Verbindungen vom Trägermaterial, dem Syntheseharz, ohne manuelles Eingreifen möglich.

Die Probenplatten sind mit einzelnen Aufnahmekammern ausgestattet, die in Ihrer Anordnung und Ausbildung mit dem Raster der Reaktionskammern in der Syntheseplatte korrespondieren. Auf diese Weise ist eine fehlerfreie und leichte Zuordnung der jeweiligen Verbindung nach ihrer Abspaltung vom Trägermaterial bzw. vom Syntheseharz gewährleistet. Eine direkte Übertragung der Reaktionsprodukte auf Hochdurchsatzscreening - Straßen wird so einfach möglich.

Der Synthesestift besitzt einen hohlzylinderförmigen Grundkörper, der durch einen Schraubverschluss verschließbar ist und ein fußseitiges Mundstück, das an die freie Öffnung der Reaktionskammern in der Syntheseplatte angepasst und mit einer Auslauföffnung ausgestattet ist. Die Auslauföffnung wird durch eine Ventilnadel mit einem Sperrventil verschlossen, die durch eine Kolbenstange und einen Kolben im Grundkörper geführt und durch eine auf den Kolben wirkende Druckfeder in ihrer Schließstellung lösbar fixiert sind. Der Zylinderraum unterhalb des Kolbens dient zur Aufnahme eines einzigen Synthesebausteines und eines Schutzgases, wobei die dosierte Abgabe des Reagenz durch einfaches Aufsetzen des Mundstückes auf dem permeablen Material, mit dem die Öffnungsseite der Reaktionskammer abgedeckt ist, erfolgt. Hierbei wird durch Eindrücken der Ventilnadel gleichzeitig das Sperrventil vom Ventilsitz gelöst und die Auslauföffnung freigegeben. Die Menge des abgegebenen Reagenz wird von der Zeitdauer bestimmt, in der das Mündstück auf dem permeablen Material aufgesetzt ist. Mit dem Abheben des Mundstückes wird die Auslauföffnung selbsttätig wieder verschlossen.

Die geschlossene Bauweise des Synthesestiftes mit einem geschlossenen Reagenzienreservoir gewährleistet eine hohe Reagenzienstabilität. Durch den Einsatz separater Synthesestifte für jeden Synthesebaustein und die öffnungsseitige Abdeckung der Reaktionskammer in den Syntheseplatten werden das Kontaminationsrisiko wesentlich gesenkt und Kreuzkontaminationen praktisch ausgeschlossen. Eine Verschleppung von Synthesebausteinen, wie sie häufig bei nicht ausreichenden Spülvorgängen auftraten, ist nicht mehr möglich.
Mit dem Fortfall der Spülvorgänge wird nicht nur der Verbrauch an organischen Lösungsmitteln erheblich gesenkt, sondern auch eine vielfach beschleunigte Synthese erreicht.

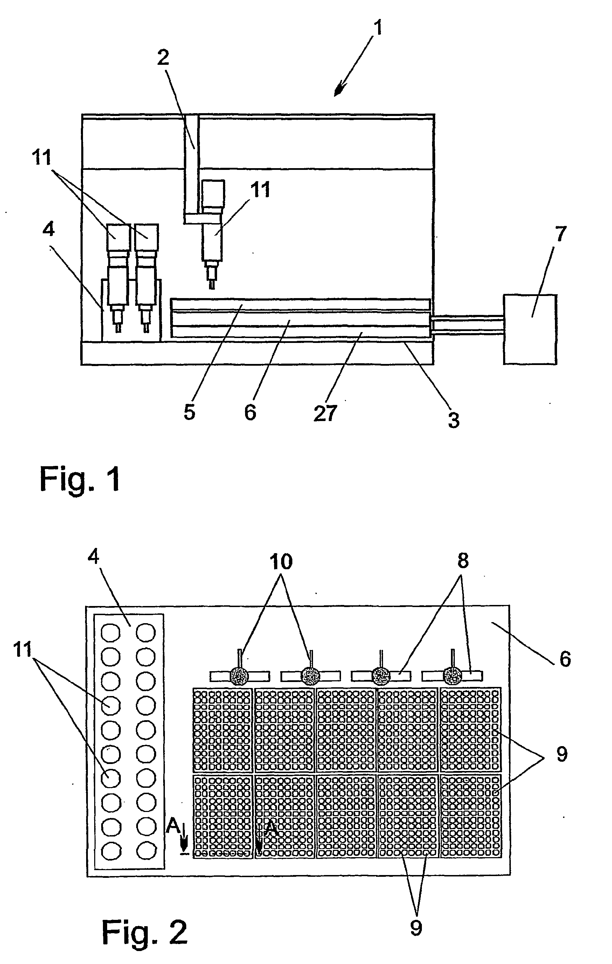

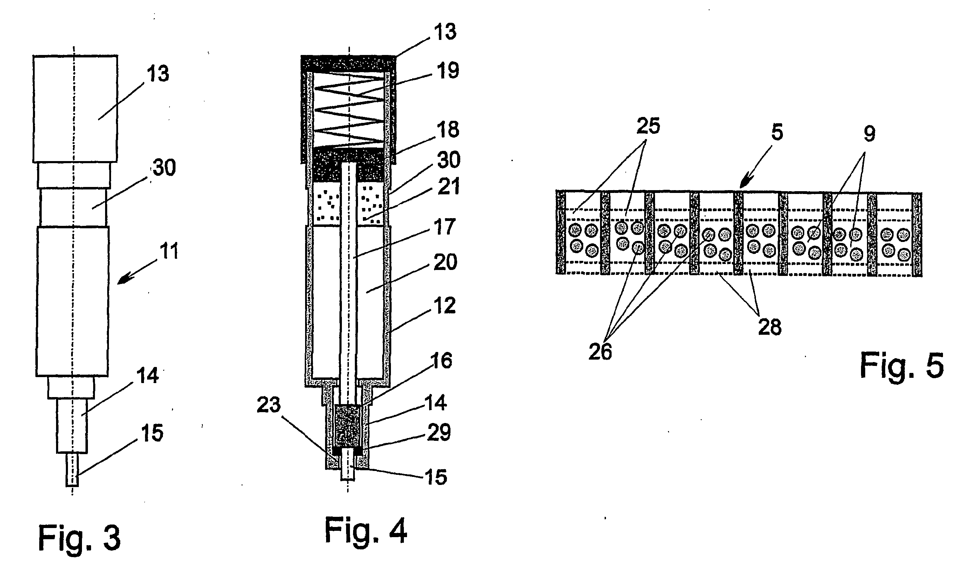

Die Erfindung soll nachstehend an einem Ausführungsbeispiel näher erläutert werden. In den dazugehörigen Zeichnungen zeigen:
- Fig.: 1: eine schematische Darstellung der erfindungsgemäßen Vorrichtung für die Synthese von Verbindungen,
- Fig.: 2: die Draufsicht auf die Arbeitsfläche der Vorrichtung nach Fig. 1,
- Fig.: 3: eine schematische Darstellung des Synthesestiftes für die separate Zuführung, Dosierung und Reagenzienbevorratung,
- Fig.: 4: einen Längsschnitt durch den Synthesestift nach Fig. 3,
- Fig.: 5: den Schnitt A - A aus Fig. 2 durch eine Syntheseplatte mit den nach der Erfindung ausgebildeten Reaktionskammern.

Die in Fig. 1 schematisch dargestellte Synthesevorrichtung 1 ist auf der Basis eines Laborpipettierroboters aufgebaut und besitzt einen in der x-, y- und z-Achse bewegbaren Greifarm 2. Auf der Arbeitsfläche 3 befinden sich Syntheseplatten 5, die bezüglich des Rasters und der Anordnung der Reaktionskammern 9 von an sich bekannten Mikrotiterplatten abgeleitet sind und ein 96er, 384er oder 1536er Raster der Reaktionskammern 9 besitzen, wodurch eine hohe Parallelisierung der Synthese erreicht wird. Die Syntheseplatten 5 sind auf einen Ventilblock 6 aufgesetzt und besitzen bodenseitig eine Membran 28 aus einem porösen Material, um die verbrauchten Reagenzien und Spülflüssigkeiten aus den Reaktionskammern 9 mit Hilfe des Ventilblockes 6, der an eine Absaugpumpe angeschlossen ist, in einen Abfall abzusaugen.

Die Synthesevorrichtung 1 ist ferner mit mehreren Spülkämmen 8 ausgestattet, die über den Spülmittelzuführungen 10 mit dem entsprechenden Spülmittelreservoir verbunden sind. Um die in den Reaktionskammern 9 befindlichen Proben, an denen ein Synthesebaustein angekuppelt worden ist, nach Ablauf der Reaktionszeit und Absaugen der verbrauchten Reaktionslösung zu spülen, wird der Spülkamm 8 mit dem benötigten Spülmittel vom Greifarm 2 aufgenommen und zur dosierten Abgabe der Spülflüssigkeit über die Reaktionskammern 9 der Syntheseplatten 5 verfahren. Nach dem Spülen wird mit einem weiteren Spülkamm 8 die zur Abspaltung der temporären Schutzgruppe des angekoppelten Synthesebausteins notwendige Lösung, wie vorstehend beschrieben, zugeführt. Nach Ablauf einer Inkubationszeit wird die Abspaltlösung über den Ventilblock 6 mit Hilfe der Absaugpumpe 7 abgezogen und die Probe gewaschen. Nach dem Waschen startet ein neuer Synthesezyklus, mit dem ein weiterer Synthesebaustein angekuppelt wird.

Nach der vorliegenden Erfindung sind für jeden Synthesebaustein separate Synthesestifte 11 vorgesehen, in denen die Reagenzien 20 in einem geschlossenen Raum vorgelegt werden und mit einem Schutzgas 21 überschichtet werden können. Die einzelnen Synthesestifte 11 mit dem jeweiligen Synthesebaustein werden in einer Halterung 4 der Synthesevorrichtung 1 bereitgestellt und mit dem Greifarm 2, der die Synthesestifte 11 an der Greifarmaufnahme 30 erfasst, zur dosierten Abgabe der Reagenzien zu den Reaktionskammer 9 der Syntheseplatten 5 gebracht.

Der erfindungsgemäß verwendete Synthesestift 11 besteht aus einem hohlzylinderförmigen Grundkörper 12 mit einem fußseitigen Mundstück 14 und einem Schraubverschluss 13, der den Zylinderraum dicht verschließt. Im Mundstück 14 befindet sich eine Auslauföffnung, die durch eine Ventilnadel 15 und ein Sperrventil 16, das in Schließposition auf einer Dichtung 29 aufliegt, verschlossen ist. Ventilnadel 15 und Sperrventil 16 werden über eine Kolbenstange 17 durch einen Kolben 18 geführt. Der erforderliche Schließdruck für das Sperrventil 16 wird durch eine Druckfeder 19 erzeugt, die auf dem Kolben 18 aufliegt und sich gegen die innere Stirnfläche des Schraubverschlusses 13 abstützt. Der freie Raum unterhalb des Kolbens 18 dient für die Vorlage des jeweiligen Synthesebausteines 20, der vorteilhafterweise mit einem Schutzgas 21 überschichtet wird. So können hochreaktive Reagenzien unter einer Schutzgasatmosphäre über lange Zeiträume stabil gehalten werden, was die Qualität der Syntheseprodukte deutlich verbessert.

Um Kreuzkontaminationen bei einem direkten Kontakt des Mundstückes 14 mit der Probe sicher auszuschließen, sind erfindungsgemäß die Reaktionskammern 9, in denen sich die Proben resp. die Festphase 26, beispielweise ein Syntheseharz befinden, öffnungsseitig mit einem permeablen Material 25, beispielsweise einer Fritte, abgedeckt. Zum Ankoppeln eines Synthesebausteines an die Probe resp. an das Syntheseharz wird das Mundstück 14 des Synthesestiftes 11 auf das die Reaktionskammer abschließende permeable Material 25 aufgesetzt, wodurch sich die Ventilnadel 15 entgegen dem Schließdruck der Druckfeder 19 nach innen verschiebt und das Sperrventil 16 freigegeben wird. Hiernach kann die Reagenzienlösung frei ausfließen, wobei die Dosierung der ausfließenden Lösung von der Zeitdauer bestimmt wird, in der das Mundstück 14 auf das Material 25 aufgesetzt ist.

Mit der Abspaltung der letzten temporären Schutzgruppe und Waschen der Proben erfolgt die Abspaltung der an die Festphase 26 angekoppelten Synthesebausteine. Hierzu wird mittels Spülkamm 8 eine Abspaltlösung zu den Proben gegeben und eine Abspaltreaktion eingeleitet. Nach Ablauf der Inkubationszeit wird der Ventilblock 6 so geschaltet, dass die in der Abspaltlösung gelösten Verbindungen in die Aufnahmekammern einer Probenplatte 27 geleitet werden, die gemäß einem weiteren Merkmal der Erfindung unter dem Ventilblock 6 angeordnet und mit einer Absauganlage verbunden ist. Die Probenplatten 27 entsprechen hinsichtlich ihres konstruktiven Aufbaus und ihrer Ausbildung den Syntheseplatten 5. Mit der Überführung der von der Festphase 26 gelösten Verbindungen in die Probenplatte 27 ist die Synthese abgeschlossen.

### Bezugszeichenliste

- 1: Synthesevorrichtung
- 2: Greifarm
- 3: Arbeitsfläche
- 4: Halterung
- 5: Syntheseplatte
- 6: Ventilblock
- 7: Absaugpumpe
- 8: Spülkamm
- 9: Reaktionskammern
- 10: Spülmittelzuführung
- 11: Synthesestift
- 12: Grundkörper
- 13: Schraubverschluss
- 14: Mundstück
- 15: Ventilnadel
- 16: Sperrventil
- 17: Kolbenstange
- 18: Kolben
- 19: Druckfeder
- 20: Synthesebaustein
- 21: Schutzgas
- 22: -
- 23: Auslauföffnung
- 24: -
- 25: permeables Material/Fritte
- 26: Festphase
- 27: Probenplatte
- 28: Membran
- 29: Dichtung
- 30: Greifarmaufnahme

## Patentansprüche

1. Vorrichtung zur gleichzeitigen multiplen und hochparallelen Synthese von Peptiden und anderen Molekülen auf der Basis der Festphasensynthese unter Verwendung eines Standardsyntheseautomaten, der einen in der x-, y- und z-Achse bewegbaren Greifarm, einen Ventilblock zur Aufnahme und Ableitung der Spülmittellösungen und mindestens einen über die auf der Arbeitsfläche des Automaten befindlichen Syntheseplatten verfahrbaren Spülkamm aufweist, der mit einem Lösungsmittelvorrat verbunden ist, **dadurch gekennzeichnet, dass** für die Synthesebausteine jeweils separate Synthesestifte (11) mit einem Reagenzienreservoir (20; 21) vorgesehen sind, die in einer gesonderten Halterung (4) für die Synthese bereitgestellt und aus dieser Halterung vom Greifarm (2) des Automaten (1) aufgenommen werden, während die Reaktionskammern (9) der Syntheseplatten (5) öffnungsseitig mit einem permeablen Material (25) verschlossen sind und unterhalb des Ventilblockes (6) eine Probenplatte (27) für die Aufnahme der nach Abspaltreaktion gelösten Proben angeordnet ist.

2. Vorrichtung nach Anspruch 1, **dadurch gekennzeichnet, dass** der Synthesestift (11) aus einem hohlzylinderförmigen Grundkörper (12) mit einem Schraubverschluss (13) und einem fußseitigen Mundstück (14) besteht und im Mundstück (14) eine Auslauföffnung (23) vorgesehen ist, die durch eine Ventilnadel (15) mit einem Sperrventil (16) verschlossen wird, welche über eine Kolbenstange (17) und einen Koben (18) geführt und durch eine auf den Kolben (18) wirkende Druckfeder (19) in ihrer Schließstellung lösbar fixiert sind, wobei der Zylinderraum unterhalb des Kolbens (18) zur Aufnahme eines Synthesebausteines (20) und eines Schutzgases (21) vorgesehen ist.

3. Vorrichtung nach Anspruch 1, **dadurch gekennzeichnet, dass** die Synthesestifte (11) durch Nanodispenser mit einem Vorratsbehälter ersetzt sind.

4. Vorrichtung nach Anspruch 1, **dadurch gekennzeichnet, dass** als Syntheseplatte (5) Platten mit im 96er, 384er oder 1536er Raster angeordneten Reaktionskammern (9) vorgesehen sind und die Probenplatte (27) mit Aufnahmekammern ausgestattet ist, die in ihrer Anordnung und Ausbildung mit dem Raster der Reaktionskammern (9) in der Syntheseplatte (5) korrespondieren.

5. Vorrichtung nach Anspruch 1, **dadurch gekennzeichnet, dass** die Probenplatten (27) an eine Absauganlage (7) angeschlossen sind.

6. Vorrichtung nach Anspruch 1, **dadurch gekennzeichnet, dass** das permeable Material (25) aus einem gegenüber dem Synthesebaustein und der Spülmittellösung resistenten Material, beispielsweise Fritte, besteht.

7. Vorrichtung nach Anspruch 1, **dadurch gekennzeichnet, dass** als Trägermaterial für die Synthese ein planares Material vorgesehen ist.

8. Vorrichtung nach Anspruch 1, **dadurch gekennzeichnet, dass** Greifarm (2) und Synthesestift (11) manuell steuerbar sind und der Synthesestift (11) separat einsetzbar ist.

## Claims

1. An apparatus for simultaneous multiple and highly parallel synthesis of peptides and other molecules based on the solid-phase synthesis technique and using a standard automated synthesizer comprising a gripper arm that can be moved along x, y, and z axes, a valve block for collecting and discharging rinsing solutions and at least one rinsing comb that can be moved above and across the synthesizing plates that are located on the working surface of the apparatus, said rinsing comb being connected to a solvent reservoir, **characterized in that** separate synthesizer pins (11) with a reagent reservoir (20; 21) are provided for each synthetic unit in a separated holder (4) for the synthesis from where the gripper arm (2) of the automated synthesizer (1) picks them up, and **in that** the openings of the reaction chambers (9) of the synthesis plates (5) are closed by a permeable material (25), and **in that** a sample plate (27) is placed underneath the valve block (6) to collect the dissolved samples after the separation reaction

2. The apparatus according to claim 1, **characterized in that** the synthesizer pin (11) consists of a hollow cylindrical body (12) with a screw-type cap (13) and a mouthpiece (14) on its foot, and **in that** said mouthpiece(14) comprises an outlet aperture (23) that is closed using a valve needle (15) with a check valve (16) guided by a piston rod (17) and a piston (18) and detachably fixed in its closed position by a pressure spring (19) acting on said piston (18) while the cylinder space underneath said piston (18) is designed to hold a synthetic unit (20) and an inert gas (21).

3. The apparatus according to claim 1, **characterized in that** said synthesizer pins (11) are substituted by nano-dispensers with a storage container.

4. The apparatus according to claim 1, **characterized in that** plates with a 96, 384, or 1536 grid of reaction chambers (9) are used as synthesizer plates (5), and that the sample plate (27) is equipped with holding chambers whose arrangement and shape correspond to the grid of reaction chambers (9) in the synthesizer plate (5).

5. The apparatus according to claim 1, **characterized in that** the sample plates (27) are connected to a suction system (7).

6. The apparatus according to claim 1, **characterized in that** the permeable material (25) consists of a material that is resistant to the synthetic unit and the rinsing solution, for example, of a frit.

7. The apparatus according to claim 1, **characterized in that** a planar material is used as carrier material for the synthesis.

8. The apparatus according to claim 1, **characterized in that** gripper arm (2) and synthesizer pin (11) can be controlled manually, and that said synthesizer pin (11) can be used separately.

## Revendications

1. Dispositif pour la synthèse multiple et hautement parallèle simultanée de peptides et autres molécules sur la base de la synthèse en phase solide en utilisant un appareil automatique de synthèse standard qui présente un bras de préhension mobile suivant l'axe x, y et z, un bloc de soupapes pour recevoir et dériver les solutions de produits de rinçage, et au moins un peigne de rinçage déplaçable via les plaques de synthèse se trouvant sur la surface de travail de l'appareil automatique, lequel est relié à une réserve de solvants, **caractérisé en ce que** pour les composants de synthèse sont prévus des pointes de synthèse (11) séparées respectives avec un réservoir à réactifs (20 ; 21) qui sont fournies pour la synthèse dans une monture (4) séparée et qui depuis cette monture sont reçues par le bras de préhension (2) de l'appareil automatique (1) tandis que les chambres de réaction (9) des plaques de synthèse (5) sont fermées côté ouverture par un matériau perméable (25) et, au-dessous du bloc de soupapes (6) est agencée une plaque d'échantillons (27) pour recevoir les échantillons dissous après la réaction d'élimination.

2. Dispositif selon la revendication 1, **caractérisé en ce que** la pointe de synthèse (11) est constituée par un corps de base (12) en forme de cylindre creux avec une fermeture vissée (13) et un embout (14) côté pied et dans l'embout (14) est prévu un orifice d'écoulement (23) qui est fermé par un pointeau de soupape (15) avec une valve d'arrêt (16), lesquels sont guidés via une tige de piston (17) et par un piston (18) et sont fixés de manière détachable par un ressort de pression (19) agissant sur le piston (18), la chambre cylindrique étant prévue en dessous du piston (18) pour recevoir un composant de synthèse (20) et un gaz de protection (21).

3. Dispositif selon la revendication 1, **caractérisé en ce que** les pointes de synthèse (11) sont remplacées par des nano-distributeurs avec un réservoir.

4. Dispositif selon la revendication 1, **caractérisé en ce qu'**il est prévu en tant que plaque de synthèse (5) des plaques avec des chambres de réaction (9) agencées en trames de 96, de 384 ou de 1536 et **en ce que** la plaque d'échantillon (27) est munie de chambres de réception qui, du point de vue de leur agencement et de leur réalisation, correspondent à la trame des chambres de réaction (9) dans la plaque de synthèse (5).

5. Dispositif selon la revendication 1, **caractérisé en ce que** les plaques d'échantillon (27) sont branchées à une installation d'aspiration (7).

6. Dispositif selon la revendication 1, **caractérisé en ce que** le matériau perméable (25) est constitué par un matériau résistant vis-à-vis du composant de synthèse et de la solution de rinçage, par exemple en fritte.

7. Dispositif selon la revendication 1, **caractérisé en ce qu'**il est prévu un matériau planaire en tant que matériau porteur pour la synthèse.

8. Dispositif selon la revendication 1, **caractérisé en ce que** le bras de préhension (2) et la pointe de synthèse (11) sont à commande manuelle et la pointe de synthèse (11) peut être utilisée séparément.
